Europäisches Patentamt

European Patent Office

Office européen des brevets

(19)

(11) Veröffentlichungsnummer: **0 264 028**
**A2**

(12) **EUROPÄISCHE PATENTANMELDUNG**

(21) Anmeldenummer: 87114313.7

(51) Int. Cl.⁴ **A61M 1/36**

(22) Anmeldetag: 01.10.87

(30) Priorität: 13.10.86 DE 3634845

(43) Veröffentlichungstag der Anmeldung:
**20.04.88 Patentblatt 88/16**

(84) Benannte Vertragsstaaten:
**AT BE CH DE ES FR GB GR IT LI LU NL SE**

(71) Anmelder: **Akzo N.V.**
**Postbus 186 Velperweg 76**
**NL-6800 LS Arnhem(NL)**

(72) Erfinder: **Baurmeister, Ulrich, Dr.**
**Moltkestrasse 67**
**D-5600 Wuppertal 1(DE)**
Erfinder: **Wollbeck, Rudi**
**Am Stadtwald 24**
**D-8765 Erlenbach/Main(DE)**

(74) Vertreter: **Fett, Günter**
**Akzo GmbH Kasinostrasse 19-23**
**D-5600 Wuppertal 1(DE)**

(54) **Kreuzstrom-Wärmeaustauscher.**

(57) Kreuzstrom-Wärmeaustauscher zur Wärmeübertragung durch Hohlfäden, deren Endbereiche in je einem mit einem Gehäusemantel fluiddicht verbundenen Vergußmasseblock eingegossen sind, bei welchem die Anschlußstutzen für das Zu- und Abführen des die Hohlfäden umströmenden Fluids in einer senkrecht zur Längsachse des Wärmeaustauschers verlaufenden Ebene derart angeordnet sind, daß ihre Längsachsen einen von 180° abweichenden Winkel miteinander bilden, vorzugsweise parallel zueinander verlaufen. Vorzugsweise beträgt die freie Länge der Hohlfäden 10 bis 40 mm, so daß der Wärmeaustauscher die Form einer Scheibe hat, ist der Gehäusemantel so gestaltet, daß er im Spritzgußverfahren herstellbar ist, bestehen die Hohlfäden aus einem biokompatiblen Material und/oder ist der Wärmeaustauscher in Reihe mit einem Blutoxygenator oder einer anderen Einrichtung zur Behandlung von Blut geschaltet.

Fig. 1

# Kreuzstrom-Wärmeaustauscher

Die Erfindung betrifft einen Kreuzstrom-Wärmeaustauscher zum Übertragen von Wärme von einem fluiden Stoff auf einen anderen fluiden Stoff durch die Wand von im wesentlichen geradlinig ausgebildeten und im wesentlichen parallel zueinander angeordneten durchgehend offenen Hohlfäden, bei welchem die Endbereiche der Hohlfäden in je einem Vergußmasseblock eingegossen sind, die beiden Vergußmasseblöcke fluiddicht mit einem die Hohlfäden umgebenden Gehäusemantel verbunden sind, der Gehäusemantel einen Anschlußstutzen für das Zuführen und einen Anschlußstutzen für das Abführen des die Hohlfäden umströmenden fluiden Stoffes aufweist und die beiden Anschlußstutzen in einer senkrecht zur Längsachse des Wärmeaustauschers verlaufenden Ebene angeordnet sind.

Ein solcher Kreuzstrom-Wärmeaustauscher ist aus der DE-OS 31 29 064 bekannt.

Unter fluidem Stoff wird im Rahmen der vorliegenden Erfindung eine Flüssigkeit, ein Flüssigkeitsgemisch, ein Dampf, ein Dampfgemisch, ein Gas, ein Gasgemisch oder ein Gemisch aus diesen Stoffen verstanden; darunter fallen beispielsweise auch Lösungen und Flüssigkeiten mit lebenden Organismen, wie beispielsweise Blut, gärende Getränke usw.

Mit der vorliegenden Erfindung wird die Aufgabe gelöst, einen Kreuzstrom-Wärmeaustauscher mit gegenüber diesem bekannten verbesserten Gebrauchseigenschaften - insbesondere im medizinischen Bereich - zur Verfügung zu stellen.

Die Erfindung besteht in einem Kreuzstrom-Wärmeaustauscher der einleitend beschriebenen Gattung, bei welchem die beiden Anschlußstutzen für den die Hohlfäden umströmenden fluiden Stoff derart angeordnet sind, daß ihre Längsachsen einen von 180° abweichenden Winkel miteinander bilden.

Diese Anordnung bewirkt in besonders vorteilhafter Weise eine Verlängerung des Strömungsweges und damit der Verweilzeit des die Hohlfäden umströmenden fluiden Stoffes im Wärmeaustauscher und demzufolge eine Erhöhung der durch die Hohlfäden übertragbaren Wärmeenergie. Diese Wirkung kann durch eine zwischen dem Einströmbereich und dem Ausströmbereich für den die Hohlfäden umströmenden fluiden Stoff angeordnete Trennwand, die einen Kurzschluß zwischen den beiden genannten Bereichen vermeiden hilft, noch verstärkt werden. Die auf diese Weise erzielbare Erhöhung der Wärmeübertragung pro Hohlfaden führt zu einer Einsparung an Hohlfadenmaterial.

Die Anordnung der beiden Anschlußstutzen ist vorzugsweise so, daß ihre Längsachsen im wesentlichen parallel zueinander verlaufen. Hierdurch wird das Anschließen einer Zuführleitung und einer Abführleitung, beispielsweise das Aufstecken von Schläuchen auf die Anschlußstutzen, erleichtert. Bei durchsichtigen Leitungen wird hierdurch die Kontrolle des Fluiddurchflusses erleichtert. Auch für die Verpackung und den Transport können sich hierdurch Vorteile ergeben, da der Raumbedarf dieser Ausführungsform des erfindungsgemäßen Wärmeaustauschers in der Regel kleiner ist.

Um einen möglichst reinen Kreuzstrom der beiden fluiden Stoffe zu erreichen, ist es zweckmäßig, die Länge des freien, d.h. des nicht eingesossenen, Abschnittes der Hohlfäden nicht zu groß zu bemessen und in einem ein bestimmtes Maß nicht übersteigenden Verhältnis zu dem hydraulischen Durchmesser der Anschlußstutzen zu bemessen. Auch sollte die Länge des freien, also des von dem einen der beiden fluiden Stoffe umströmbaren, Abschnittes der Hohlfäden im Verhältnis zum Querschnitt des Hohlfadenbündels, also dem Querschnitt des Gehäusemantels ein bestimmtes Maß nicht überschreiten. Als besonders vorteilhaft hat es sich erwiesen, wenn die Länge des nicht eingegossenen Abschnittes der Hohlfäden nicht größer ist als der hydraulische Durchmesser des Querschnitts des Gehäusemantels. Der hydraulische Durchmesser der Anschlußstutzen wiederum sollte vorzugsweise wenigstens 1/4 der Länge des freien Abschnittes der Hohlfäden betragen.

Der hydraulische Durchmesser ist eine in der Strömungslehre übliche Bezugsgröße (-länge) zur Charakterisierung eines Strömungskanals; er ist der Quotient aus vierfacher Querschnittsfläche und (benetztem) Umfang eines Strömungskanals, also $4 \cdot A/U$ (mit $A$ = Querschnittsfläche und $U$ = Umfang des Strömungskanals). Für Strömungskanäle, z.B. Rohre, mit kreisförmigem Querschnitt stimmt der hydraulische Durchmesser mit dem tatsächlichen überein, wie die einfache Nachrechnung ergibt.

Für den Einsatz im medizinischen Bereich, insbesondere zum Temperieren von Blut, hat es sich als ausreichend und besonders vorteilhaft erwiesen, wenn die Länge des freien Abschnittes der Hohlfäden lediglich 10 bis 40 mm beträgt. Daraus ergibt sich, daß der erfindungsgemäße Wärmeaustauscher vorzugsweise scheibenförmig ausgebildet ist, wobei die Anzahl der Hohlfäden 10.000 oder 20.000 oder mehr betragen kann.

Ein Vorteil der verhältnismäßig kurzen Länge der Hohlfäden des erfindungsgemäßen Wärmeaustauschers besteht darin, daß der Druckverlust in den Hohlfäden verhältnismäßig gering ist. Das bedeutet, daß Blut beispielsweise auf sehr schonende Art und Weise unter Anwendung nur eines geringen Druckes durch die Hohlfäden des erfindungsgemäßen Wärmeaustauschers gefördert werden kann. Trotzdem ist es möglich, falls gewünscht, Blut auch als den die Hohlfäden umströmenden fluiden Stoff zu verwenden.

Für den Einsatz im medizinischen Bereich ist der erfindungsgemäße Wärmeaustauscher vorzugsweise mit biokompatiblen Hohlfäden ausgestattet, beispielsweise solchen aus Polycarbonat, PVDF oder Polysulfon.

Die Wandstärke der Hohlfäden beträgt vorzugsweise 5 bis 20% ihres Innendurchmessers und liegt mit Vorteil im Bereich von 10 $\mu$m bis 120 $\mu$m. Der Innendurchmesser der Hohlfäden des erfindungsgemäßen Wärmeaustauschers liegt demnach vorzugsweise im Bereich von 200 bis 600 $\mu$m.

Die Hohlfäden des erfindungsgemäßen Wärmeaustauschers sind vorzugsweise zumindest für die an der Wärmeübertragung teilnehmenden fluiden Stoffe undurchlässig.

Die gleichmäßige Verteilung der Hohlfäden in den Vergußmasseblöcken bis in den zum Gehäusemantel hin sich erstreckenden Randbereich führt zu einer nicht unerheblichen Einsparung von Vergußmasse, bei der es sich häufig um Polyurethan handelt.

Zur Herstellung des Hohlfadenbündels wird vorzugsweise von einer Hohlfadenmatte ausgegangen. Hierbei handelt es sich um eine Hohlfadenanordnung in Gewebeform, bei welcher parallel zueinander und in vorzugsweise gleichen Abständen voneinander angeordnete Hohlfäden beispielsweise durch quer zu ihren Längsachsen verlaufende Klebestreifen oder durch mittels eines Web-oder Wirkverfahrens eingebrachte (textile) Fäden in der zuvor beschriebenen Anordnung festgehalten werden. Verfahren zum Herstellen solcher Hohlfadenmatten sowie diese selbst sind an sich bekannt. Durch Aufrollen einer solchen Matte erhält man ein Hohlfadenbündel aus im wesentlichen geradlinig und parallel zueinander angeordneten Hohlfäden, in welchem die Hohlfäden in definierten Abständen voneinander gehalten werden, wobei der gegenseitige Abstand in Umfangsrichtung ein anderer sein kann als der in radialer Richtung. Als vorteilhaft hat es sich dabei erwiesen, den seitlichen Abstand in Umfangsrichtung so zu bemessen, daß er größer ist als der Außendurchmesser der Hohlfäden.

Auf diese Weise wird eine gleichmäßige radiale Durchströmung des Hohlfadenbündels ermöglicht. Bei der Herstellung der Hohlfadenmatten wird der Abstand zwischen den obengenannten die Hohlfäden auf Abstand festhaltenden Klebestreifen oder (textilen) Fäden vorzugsweise so bemessen, daß die genannten Haltemittel im fertigeingegossenen Hohlfadenbündel entweder nicht mehr enthalten sind oder sich in den Verußmasseblöcken befinden, so daß das freie Durchströmen des Hohlfadenbündels durch sie nicht behindert wird.

Die Herstellung des Gehäusemantels geschieht vorzugsweise im Spritzgußverfahren. Die Anschlußstutzen können einen zum Gehäusemantel hin größer werdenden Querschnitt aufweisen, also diffusorähnlich ausgebildet sein. Im Bereich des Gehäusemantels können die Abmessungen der Anschlußstutzen ungefähr der Länge des nicht eingegossenen Abschnittes der Hohlfäden und der halben Breite bzw. dem halben Durchmesser des Hohlfadenbündels entsprechen.

Im Bereich der Längsachse des erfindungsgemäßen Wärmeaustauschers kann zumindest im Bereich der freien Abschnitte der Hohlfäden ein zylinderförmiger Kern angeordnet sein. Bei der Herstellung des Hohlfadenbündels aus einer Hohlfadenmatte, wie oben beschrieben, kann die Hohlfadenmatte um diesen Kern herumgewickelt werden.

Zum Zu-und Abführen des die Hohlfäden durchströmenden fluiden Stoffes ist vorzugsweise an beiden Enden des erfindungsgemäßen Wärmeaustauschers eine Kappe angeordnet, die den Verteilerraum bzw. den Sammelraum für den genannten fluiden Stoff bildet. Aus diesem Grund weisen die Endbereiche des Gehäusemantels vorzugsweise einen kleineren Umfang auf als die sich unmittelbar daran anschließenden Bereiche. Hierdurch kann das Aufsetzen der Kappen erleichtert werden. Auch die Kappen sind vorzugsweise als Spritzgußteile ausgebildet. Vorzugsweise ist der erfindungsgemäße Wärmeaustauscher mit wenigstens einer solchen Kappe versehen. Die hierfür geeigneten Kappen können einen tangential mündenden oder einen im Bereich der Längsachse der Kappe und somit auch des Wärmeaustauschers angeordneten in Längsrichtung mündenden Anschlußstutzen aufweisen.

Bei Verwendung des erfindungsgemäßen Wärmeaustauschers in der Weise, daß Blut die Hohlfäden durchströmt, hat es sich als besonders vorteilhaft erwiesen, für das gleichmäßige Verteilen des Blutes auf alle Hohlfäden eine Kappe mit einem tangential im äußeren Umfangsbereich der Kappe mündenden Anschlußstutzen und auf der gegenüberliegenden Seite, auf welcher das aus den Hohlfäden austretende Blut gesammelt und weggeführt wird, eine diffusorähnlich ausgebildete Kappe, die einen in ihrer Längsachse angeordneten in Längsrichtung mündenden Anschlußstutzen aufweist, vorzusehen.

Der erfindungsgemäße Wärmeaustauscher kann auch als eine Grundeinheit eines nach dem Baukastenprinzip aufbaubaren oder aufgebauten Systems eingesetzt werden. D.h. es können mehrere erfindungsgemäße Wärmeaustauscher durch Reihenschaltung zu einer größeren Einheit zusammengesetzt werden oder ein oder mehrere erfindungsgemäße Wärmeaustauscher - insbesondere im medizinischen Bereich - mit anderen Einrichtungen ein System bilden. Das Zusammensetzen mehrerer erfindungsgemäßer Wärmeaustauscher bzw. eines oder mehrerer erfindungsgemäßer Wärmeaustauscher mit einer oder mehreren anderen Einrichtungen in beliebiger also auch abwechselnder Reihenfolge geschieht am zweckmäßigsten in der Weise, daß die genannten Teile unter Weglassen der sonst üblichen Kappen stirnseitig so aneinandergereiht werden, daß zwischen den Hohlfadenenden benachbarter Einheiten bzw. Einrichtungen ein eine Kammer bildender Zwischenraum gelassen wird und die Gehäusemäntel mit nach außen fluiddichter Wirkung miteinander verbunden werden, beispielsweise durch einen muffenähnlichen die Gehäusemantelenden überdeckenden Ring. Eine solche Verbindung kann auch als Schraubverbindung, Schnappverschluß od.dgl. ausgebildet sein. Vorzugsweise sind die Strömungsquerschnitte benachbarter Einheiten bzw. Einrichtungen, insbesondere dann, wenn es sich bei dem die Hohlfäden durchströmenden fluiden Stoff um Blut oder eine andere Körperflüssigkeit oder schonend zu behandelnde Flüssigkeit handelt, gleich groß bemessen.

Der erfindungsgemäße Wärmeaustauscher hat sich insbesondere in Kombination mit einem Blutoxygenator bewährt, bei dem die Sauerstoffübertragung ins Blut und die Kohlendioxidentfernung aus dem Blut durch die Wand von durchgegend offenen Hohlfäden erfolgt, deren Endbereiche in je einem Vergußmasseblock eingegossen sind, die mit einem die Hohlfäden umgebenden Gehäusemantel fluiddicht verbunden sind, bei dem der Gehäusemantel wenigstens eine Öffnung für das Zu-oder Abführen des Sauerstoffs oder des Blutes aufweist, bei dem im Bereich seiner Längsachse ein über die gesamte Länge der Hohlfäden sich erstreckender Verteilerkörper für das über die gesamte freie Länge der Hohlfäden gleichmäßige Zu-oder Abführen des Sauerstoffs oder des Blutes angeordnet ist, bei welchem die Hohlfäden mikroporös sind und durchgehende nach außen offene Poren aufweisen, bei welchem die Hohlfäden über den gesamten Querschnitt der Vergußmasseblöcke bis in den zum Gehäusemantel hin sich erstreckenden Randbereich gleichmäßig verteilt angeordnet sind und bei welchem zwischen dem nicht eingegossenen Bereich der äußeren Hohlfäden und dem Gehäusemantel wenigstens ein freier Raum mit ringförmigem Querschnitt vorhanden ist, in den die wenigstens eine Öffnung mündet, wie er in der (P ................) (internes Aktenzeichen A3GW32160) beschrieben ist, und der in der oben beschriebenen Weise, d.h. mit einer muffenähnlichen Verbindung, mit dem erfindungsgemäßen Wärmeaustauscher zusammengekoppelt ist.

Auch bei einer Reihenschaltung des erfindungsgemäßen Wärmeaustauschers mit einem solchen Blutoxygenator gilt, daß es vorteilhaft sein kann, für das Zuführen des die Hohlfäden durchströmenden Blutes eine Kappe mit tangential mündendem Anschlußstutzen und für das Abführen des aus den Hohlfäden ausgetretenen Blutes eine diffusorähnlich ausgebildete Kappe zu verwenden, wobei auch hierbei in besonders vorteilhafter Weise die Blutströmungsquerschnitte der beiden in Reihe geschalteten Teile gleich groß bemessen sind und Verengungen oder Erweiterungen der blutführenden Teile zwischen den genannten Teilen nicht vorhanden sind.

Die Erfindung wird nun anhand der Zeichnung näher erläutert. In vereinfachter teilweise geschnittener bzw. schematischer Darstellungsweise zeigt:

Fig. 1 einen Wärmeaustauscher in Seitenansicht,

Fig. 2 den Wärmeaustauscher nach Fig. 1 in Draufsicht,

Fig. 3 den Wärmeaustauscher nach Fig. 1 und 2 in Reihenschaltung mit einem Blutoxygenator,

Fig. 4 den Wärmeaustauscher nach Fig. 1 und 2 mit unterschiedlichen Kappen,

Fig. 5 einen Wärmeaustauscher mit diffusorähnlich ausgebildeten Anschlußstutzen.

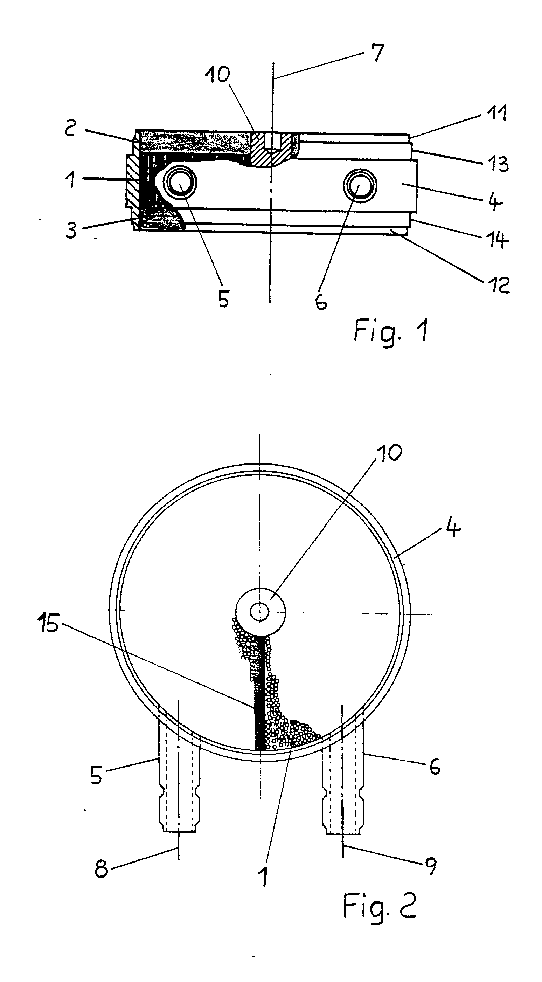

Der in den Fig. 1 und 2 dargestellte erfindungsgemäße Kreuzstrom-Wärmeaustauscher weist folgende Teile auf: Eine Vielzahl (ein Bündel) im wesentlichen parallel zueinander angeordneter durchgehend offener Hohlfäden 1, deren Endbereiche in je einem Vergußmasseblock 2 bzw. 3 eingegossen sind, die Vergußmasseblöcke 2 und 3, die fluiddicht mit dem die Hohlfäden 1 umgebenden Gehäusemantel 4 verbunden sind, sowie die beiden Anschlußstutzen 5 und 6, von denen einer für das Zuführen und einer für das Abführen des die Hohlfäden 1 umströmenden fluiden Stoffes vorgesehen ist, wobei die beiden Anschlußstutzen 5 und 6 in einer senkrecht zur Längsachse 7 des Wärmeaustauschers verlaufenden Ebene angeordnet sind, und zwar derart, daß die Längsachsen 8 und 9 der Anschlußstutzen 5 bzw. 6 im wesentlichen parallel zueinander verlaufen. Im Bereich der Längsachse 7 des Wärmeaustauschers ist der zylinderförmige Kern 10 angeordnet, dessen Länge der Gesamtlänge der Hohlfäden 1 und nicht nur der Länge der freien, nicht eingegossenen Abschnitte der Hohlfäden 1 entspricht. Die Endbereiche 11 und 12 des Gehäusemantels 4 weisen einen kleineren Umfang auf als die sich unmittelbar daran anschließenden Bereiche 13 bzw. 14. Die Hohlfäden 1 sind über den gesamten Querschnitt der Vergußmasseblöcke 2 und 3 bis in den zum Gehäusemantel 4 hin sich erstreckenden Randbereich gleichmäßig verteilt angeordnet. Der Gehäusemantel 4 ist so ausgebildet, daß er zusammen mit den Anschlußstutzen 5 und 6 als ein komplettes Teil im Spritzgußverfahren hergestellt werden kann. Wie sich aus Fig. 2 ergibt, bewirkt die dort gezeigte Anordnung der Anschlußstutzen 5 und 6 eine beträchtliche Verlängerung des Strömungsweges und damit der Verweilzeit des die Hohlfäden 1 umströmenden fluiden Stoffes im Wärmeaustauscher. Durch die zwischen dem Einströmbereich und dem Auströmbereich für den die Hohlfäden 1 umströmenden fluiden Stoff angeordnete Trennwand 15 wird ein Kurzschluß zwischen den beiden genannten Bereichen vermieden und die zuvor geschilderte Wirkung damit noch verstärkt. Wie die Figuren 1 und 2 darüber hinaus deutlich zeigen, ist bei dieser Ausführungsform die Länge des freien, nicht eingegossenen Abschnittes jedes Hohlfadens 1 kleiner als der Durchmesser des Hohlfadenbündels 1 und somit auch des Gehäusemantels 4, so daß der Wärmeaustauscher scheibenförmig ausgebildet ist.

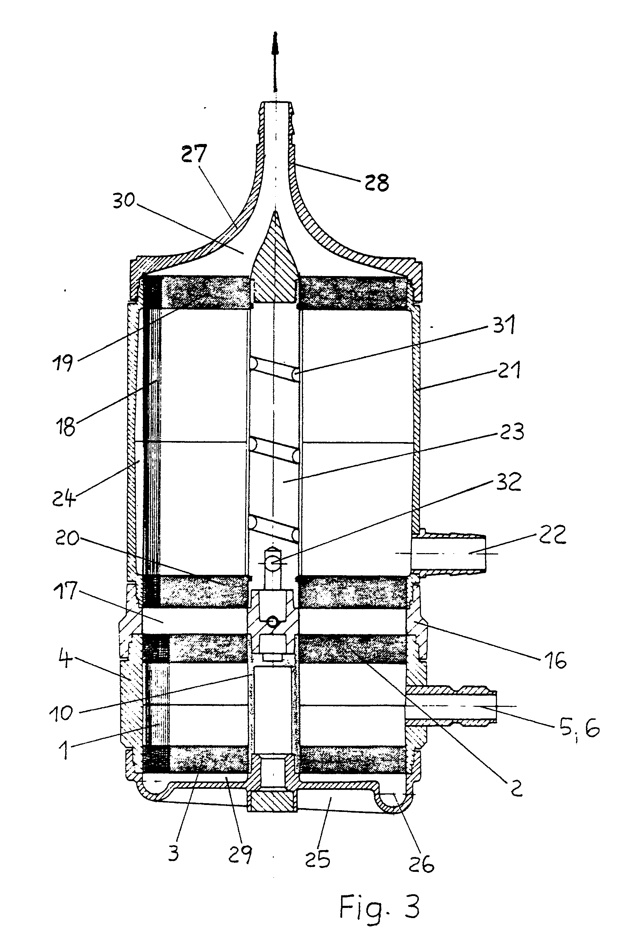

In Fig. 3 ist der Wärmeaustauscher nach Fig. 1 und 2 zusammen mit einem Blutoxygenator dargestellt, mit welchem er über den muffenähnlichen Verbindungsring 16 zusammengekoppelt und somit blutseitig in Reihe geschaltet ist. Gegenüber der in Fig. 1 gezeigten Position ist der Wärmeaustauscher in Fig. 3 um 90° gedreht, so daß nur einer der beiden Anschlußstutzen 5 und 6 erkennbar ist. Zwischen den Hohlfadenenden des Wärmeaustauschers und denen des Oxygenators ist ein die Kammer 17 bildender Zwischenraum belassen. Der Blutströmungsquerschnitt des Wärmeaustauschers und der des Oxygenators sind gleich groß bemessen, und die Kammer 17 ist so ausgebildet, daß Verengungen oder Erweiterungen des durch die Kammer 17 gebildeten blutführenden ·Kanals nicht vorhanden sind. Der Blutoxygenator besteht aus den die Sauerstoffübertragung ins Blut und die Kohlendioxidentfernung aus dem Blut durch ihre Wand bewirkenden durchgehend offenen Hohlfäden 18, deren Endbereiche in je einem Vergußmasseblock 19 bzw. 20 eingegossen sind, und dem Gehäusemantel 21, mit dem die Vergußmasseblöcke 19 und 20 fluiddicht verbunden sind; der Gehäusemantel 21 weist den Anschlußstutzen 22 für das Zu-oder Abführen des Sauerstoffs auf; im Bereich seiner Längsachse weist der Blutoxygenator den über die gesamte Länge seiner Hohlfäden 18 sich erstreckenden Verteilerkörper 23 für das über die gesamte freie Länge der Hohlfäden 18 gleichmäßige Ab-oder Zuführen des Sauerstoffs auf. Das Ab-bzw. Zuführen des Sauerstoffs erfolgt dabei über den auf der Außenseite des Verteilerkörpers 23 angeordneten spiralförmig verlaufenden Kanal 31, der in den Kanal 32 mündet, der nach außen geführt ist. Die Hohlfäden 18 sind mikroporös und weisen durchgehende nach außen offene Poren auf. Auch die Hohlfäden 18 sind über den gesamten Querschnitt der Vergußmasseblöcke 19 und 20 bis in den zum Gehäusemantel 21 hin sich erstreckenden Randbereich gleichmäßig verteilt angeordnet, wobei der Gehäusemantel 21 so ausgestaltet ist, daß zwischen den äußeren Hohlfäden 18 und dem Gehäusemantel 21 der freie Raum 24 mit ringförmigem Querschnitt gebildet wird, in welchen der Anschlußstutzen 22 mündet. Für das Zuführen des die Hohlfäden durchströmenden Blutes zum Wärmeaustauscher ist die Kappe 25 mit dem im Bereich des äußeren Umfangs des Hohlfadenbündels 1 des Wärmeaustauschers mündenden Anschlußstutzen 26 angeordnet. Zum Abführen des aus den Hohlfäden 18 des Oxygenators ausgetretenen Blutes ist die diffusorähnlich ausgebildete Kappe 27 mit dem im Bereich ihrer Längsachse angeordneten in Längsrichtung mündenden Anschlußstutzen 28 angeordnet. Die Kappe 25 bildet den Verteilerraum 29 für das die Hohlfäden 1 durchströmende Blut, während die Kappe 27 den Sammelraum 30 für das aus den Hohlfäden 18 austretende Blut bildet. Die Kammer 17 wirkt sowohl als Sammelraum für das aus den Hohlfäden 1 austretende Blut als auch als Verteilerraum für das in die Hohlfäden 18 eintretende Blut.

Die in Fig. 3 dargestellte Anordnung und Ausgestaltung von Wärmeaustauscher und Blutoxygenator kann in besonders vorteilhafter Weise wie dargestellt mit der flachen Kappe 25 nach unten auf jede beliebige im wesentlichen waagerechte ebene Fläche gestellt werden, da es irgendwelcher Einrichtungen zum Befestigen oder Aufhängen der Vorrichtung dabei nicht bedarf. Bei dieser Anordnung ergibt sich als weiterer Vorteil, daß eine Befüllung der Hohlfäden 1 bzw. 18 von unten nach oben erfolgt und damit ein gleichmäßiges Verdrängen des in dem Verteilerraum 29, den Hohlfäden 1, der Kammer 17, den Hohlfäden 18 und dem Sammelraum 30 enthaltenen Gases, das sogenannte Entlüften, stattfindet.

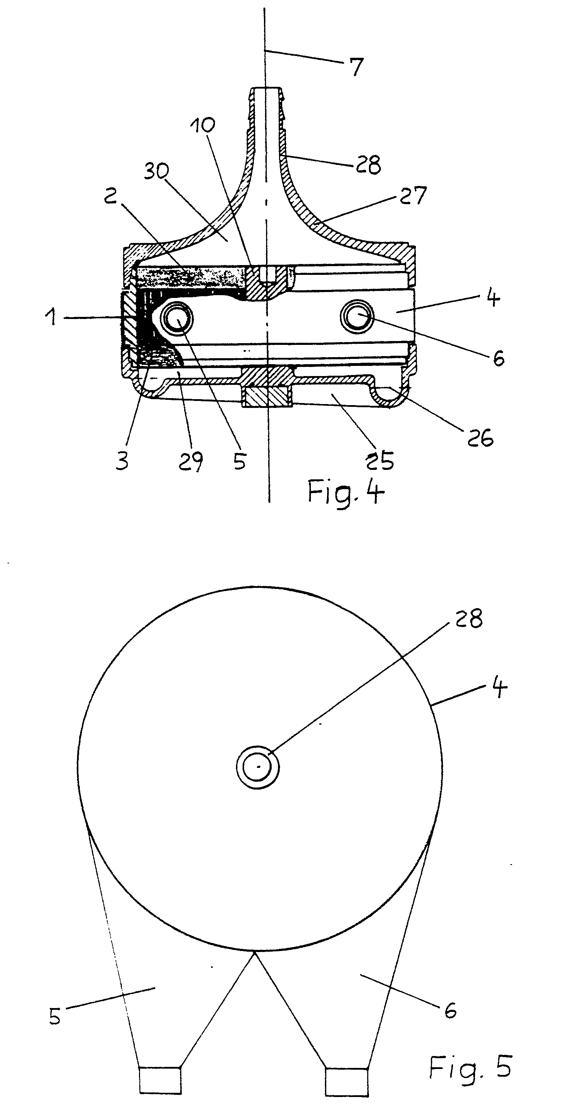

In Figur 4 ist der Wärmeaustauscher nach den Figuren 1 und 2 dargestellt, wobei bei dieser Ausführungsform der Wärmeaustauscher mit einer Kappe 25 und einer Kappe 27, wie sie in Figur 3 dargestellt und weiter oben ausführlich beschrieben sind, versehen ist. Die übrigen Teile ergeben sich ihren Positionszahlen entsprechend aus der Beschreibung der Figuren 1 bis 3.

In Ausgestaltung der in den Figuren 3 und 4 dargestellten Ausführungsformen können statt unterschiedlicher auch gleiche Kappen verwendet werden, daß heißt beispielsweise entweder zwei Kappen entsprechend der Kappe 25 oder zwei Kappen entsprechend der Kappe 27. In weiterer Ausgestaltung kann auch wenigstens eine Kappe verwendet werden, bei der der Anschlußstutzen für das die Hohlfäden durchströmende Fluid im Bereich der Längsachse des Wärmeaustauschers bzw. des Blutoxigenators tangential in den Verteiler bzw. Sammelraum mündet.

In Figur 5 ist eine Ausführungsform des Wärmeaustauschers mit zum Gehäusemantel 4 hin sich erweiternden Anschlußstutzen 5 und 6 dargestellt. Bei rundem Querschnitt der Anschlußstutzen 5 und 6 entspricht dies einer diffusorähnlichen Ausgestaltung. Bei einer im wesentlichen scheibenförmigen Ausgestaltung des Wärmeaustauschers kann die Höhe der Anschlußstutzen 5 und 6 der Länge des freien, nicht eingegossenen Abschnitts der Hohlfäden entsprechend bemessen sein, so daß sich ein rechteckiger Strömungsquerschnitt ergibt, der eine äußerst gleichmäßige Verteilung des die Hohlfäden umströmenden Fluids auf alle Hohlfäden bewirkt.

Zwischen der in den Figuren 1 bis 4 dargestellten Ausführungsform der Anschlußstutzen 5 und 6 und der in Figur 5 dargestellten liegende Formen der Anschlußstutzen 5 und 6 können nach Bedarf zur weiteren vorteilhaften Ausgestaltung des erfindungsgemäßen Wärmeaustauschers gewählt werden.

Beispiel:

Ein Wärmeaustauscher, der sich in Reihenschaltung mit einem Blutoxygenator (ähnlich Fig. 3) gut bewährt hat, weist folgende Merkmale auf:

Anzahl der Hohlfäden:     15 680
Länge der Hohlfäden:     40 mm
Freie, nicht eingegossene Länge der Hohlfäden: 28 mm
Innendurchmesser der Hohlfäden:    380 $\mu$m
Wandstärke der Hohlfäden:    50 $\mu$m
Durchmesser des Hohlfadenbündels:    93,5 mm
Innendurchmesser des Gehäusemantels:    93,5 mm
Durchmesser des Kerns:    18 mm
Innendurchmesser der Anschlußstutzen:    9,6 mm
Füllgrad des Hohlfadenbündels:    42,9 %

## Ansprüche

1. Kreuzstrom-Wärmeaustauscher zum Übertragen von Wärme von einem fluiden Stoff auf einen anderen fluiden Stoff durch die Wand von im wesentlichen geradlinig ausgebildeten und im wesentlichen parallel zueinander angeordneten durchgehend offenen Hohlfäden, bei welchem die Endbereiche der Hohlfäden in je einem Vergußmasseblock eingegossen sind, die beiden Vergußmasseblöcke fluiddicht mit einem die Hohlfäden umgebenden Gehäusemantel verbunden sind, der Gehäusemantel einen Anschlußstutzen für das Zuführen und einen Anschlußstutzen für das Abführen des die Hohlfäden umströmenden fluiden Stoffes aufweist und die beiden Anschlußstutzen in einer senkrecht zur Längsachse des Wärmeaustauschers verlaufenden Ebene angeordnet sind, dadurch gekennzeichnet, daß die beiden Anschlußstutzen derart angeordnet sind, daß ihre Längsachsen einen von 180° abweichenden Winkel miteinander bilden.

2. Wärmeaustauscher nach Anspruch 1, dadurch gekennzeichnet, daß die Längsachsen der Anschlußstutzen einen Winkel von höchstens 90° miteinander bilden.

3. Wärmeaustauscher nach Anspruch 1 oder 2, dadurch gekennzeichnet, daß die Längsachsen der Anschlußstutzen im wesentlichen parallel zueinander verlaufen.

4. Wärmeaustauscher nach einem der Ansprüche 1 bis 3, dadurch gekennzeichnet, daß die Länge des freien (nicht eingegossenen) Abschnittes der Hohlfäden nicht größer ist als der hydraulische Durchmesser des Querschnitts des Gehäusemantels.

5. Wärmeaustauscher nach einem der Ansprüche 1 bis 4, dadurch gekennzeichnet, daß die Hohlfäden über den gesamten Querschnitt der Vergußmasseblöcke bis in den zum Gehäusemantel hin sich erstreckenden Randbereich gleichmäßig verteilt angeordnet sind.

6. Wärmeaustauscher nach einem der Ansprüche 1 bis 5, dadurch gekennzeichnet, daß der hydraulische Durchmesser der Anschlußstutzen wenigstens 1/4 der Länge des freien (nicht eingegossenen) Abschnittes der Hohlfäden beträgt.

7. Wärmeaustauscher nach einem der Ansprüche 1 bis 6, dadurch gekennzeichnet, daß die Länge des freien (nicht eingegossenen)Abschnittes der Hohlfäden 10 bis 40 mm beträgt.

8. Wärmeaustauscher nach einem der Ansprüche 1 bis 7, dadurch gekennzeichnet, daß der Querschnitt der Anschlußstutzen zum Gehäusemantel hin größer wird.

9. Wärmeaustauscher nach einem der Ansprüche 1 bis 8, dadurch gekennzeichnet, daß der hydraulische Durchmesser der Anschlußstutzen im Bereich des Gehäusemantels gleich der Länge des freien (nicht eingegossenen) Abschnittes der Hohlfäden ist.

10. Wärmeaustauscher nach einem der Ansprüche 1 bis 9, gekennzeichnet durch einen in seinem Längsachsenbereich zumindest im Bereich der freien (nicht eingegossenen) Abschnitte der Hohlfäden angeordneten zylinderförmigen Kern.

11. Wärmeaustauscher nach einem der Ansprüche 1 bis 10, dadurch gekennzeichnet, daß die Hohlfäden aus einem vollsynthetischen Polymeren bestehen.

12. Wärmeaustauscher nach einem der Ansprüche 1 bis 11, dadurch gekennzeichnet, daß die Hohlfäden aus einem biokompatiblen Material bestehen.

13. Wärmeaustauscher nach einem der Ansprüche 1 bis 12, dadurch gekennzeichnet, daß die Endbereiche des Gehäusemantels einen kleineren Umfang aufweisen als die sich unmittelbar daran anschließenden Bereiche.

14. Wärmeaustauscher nach einem der Ansprüche 1 bis 13, dadurch gekennzeichnet, daß der Gehäusemantel als Spritzgußteil ausgebildet ist.

15. Vorrichtung nach einem der Ansprüche 1 bis 14, gekennzeichnet durch eine an wenigstens einem Ende aufgesetzte einen Verteiler-bzw. Sammelraum für den die Hohlfäden durchströmenden fluiden Stoff bildende Kappe mit einem tangential mündenden Anschlußstutzen.

16. Wärmeaustauscher nach einem der Ansprüche 1 bis 15, gekennzeichnet durch eine an wenigstens einem Ende aufgesetzte einen Verteiler-bzw. Sammelraum für den die Hohlfäden durchströmenden fluiden Stoff bildende diffusorähnlich ausgebildete Kappe mit einem im Bereich ihrer Längsachse angeordneten in Längsrichtung mündenden Anschlußstutzen.

17. Verwendung des Wärmeaustauschers nach einem der Ansprüche 1 bis 16 zum Temperieren von Blut.

18. Verwendung des Wärmeaustauschers nach einem der Ansprüche 1 bis 16 in einer Einrichtung zur Behandlung von Blut.

19. Wärmeaustauscher nach einem der Ansprüche 1 bis 16, dadurch gekennzeichnet, daß er in Reihe mit einem Blutoxygenator geschaltet ist.

20. Wärmeaustauscher nach Anspruch 19, dadurch gekennzeichnet, daß die Blutströmungsquerschnitte des Wärmeaustauschers und des Blutoxygenators gleich groß bemessen sind.

21. Wärmeaustauscher nach einem der Ansprüche 1 bis 16, 19 oder 20, dadurch gekennzeichnet, daß er zum Temperieren von Blut vorgesehen ist und daß für das Zuführen des Blutes an einem Ende eine den Verteilerraum für das die Hohlfäden durchströmende Blut bildende Kappe mit einem tangential mündenden Anschlußstutzen und für das Abführen des Blutes am anderen Ende eine den Sammelraum für das aus den Hohlfäden austretende Blut bildende diffusorähnlich ausgebildete Kappe mit einem im Bereich ihrer Längsachse angeordneten in Längsrichtung mündenden Anschlußstutzen angeordnet ist.

22. Wärmeaustauscher nach einem der Ansprüche 1 bis 16 oder 20, dadurch gekennzeichnet, daß er in Reihe mit einem Blutoxygenator geschaltet ist, bei dem die Sauerstoffübertragung ins Blut und die Kohlendioxidentfernung aus dem Blut durch die Wand von durchgehend offenen Hohlfäden erfolgt, deren Endbereiche in je einem Vergußmasseblock eingegossen sind, die mit einem die Hohlfäden umgebenden Gehäusemantel fluiddicht verbunden sind, bei dem der Gehäusemantel wenigstens eine Öffnung für das Zu-oder Abführen des Sauerstoffs oder des Blutes aufweist, bei dem im Bereich seiner Längsachse ein über die gesamte Länge der Hohlfäden sich erstreckender Verteilerkörper für das über die gesamte freie Länge der Hohlfäden gleichmäßige Zu-oder Abführen des Sauerstoffs oder des Blutes angeordnet ist, bei welchem die Hohlfäden mikroporös sind und durchgehende nach außen offene Poren aufweisen, bei welchem die Hohlfäden über den gesamten Querschnitt der Vergußmasseblöcke bis in den zum Gehäusemantel hin sich erstreckenden Randbereich gleichmäßig verteilt angeordnet sind und bei welchem zwischen dem nicht eingegossenen Bereich der äußeren Hohlfäden und dem Gehäusemantel wenigstens ein freier Raum mit ringförmigem Querschnitt vorhanden ist, in den die wenigstens eine Öffnung mündet, daß der Blutoxygenator mit einer muffenähnlichen Verbindung mit dem erfindungsgemäßen Wärmeaustauscher zusammengekoppelt ist und daß für das Zuführen des die Hohlfäden durchströmenden Blutes eine Kappe mit tangential mündendem Anschlußstutzen und für das Abführen des aus den Hohlfäden ausgetretenen Blutes eine diffusorähnlich ausgebildete Kappe angeordnet ist.

23. Wärmeaustauscher nach einem der Ansprüche 1 bis 16 oder 19 bis 22, gekennzeichnet durch eine zwischen dem Einströmbereich und dem Ausströmbereich für den die Hohlfäden umströmenden fluiden Stoff angeordnete Trennwand.

Fig. 1

Fig. 2

A3GW32161

Fig. 3

0 264 028

A3GW32161

Fig. 4

Fig. 5